(19) Europäisches Patentamt
European Patent Office
Office européen des brevets

(11) Numéro de publication: **0 263 861**
**B1**

(12) # FASCICULE DE BREVET EUROPEEN

(45) Date de publication du fascicule du brevet: **01.08.90**

(21) Numéro de dépôt: **87902538.5**

(22) Date de dépôt: **07.04.87**

(86) Numéro de dépôt international:
**PCT/FR87/00114**

(87) Numéro de publication internationale:
**WO 87/06229 22.10.87 Gazette 87/23**

(51) Int. Cl.⁵: **C 07 D 257/02**, A 61 K 49/00, C 07 F 5/00, C 07 C 229/26

(54) **SEL DE LYSINE DU COMPLEXE GADOLINIUM-DOTA ET SES APPLICATIONS AU DIAGNOSTIC.**

(30) Priorité: **11.04.86 FR 8605235**

(43) Date de publication de la demande:
**20.04.88 Bulletin 88/16**

(45) Mention de la délivrance du brevet:
**01.08.90 Bulletin 90/31**

(84) Etats contractants désignés:
**AT BE CH DE FR GB IT LI LU NL SE**

(56) Documents cités:
**FR-A-2 539 996**

(73) Titulaire: **GUERBET S.A.**
**16-24 rue Jean Chaptal**
**F-93609 Aulnay sous Bois Cédex (FR)**

(72) Inventeur: **BONNEMAIN, Bruno**
**9, rue de Reims**
**F-77290 Mitry Mory (FR)**
Inventeur: **LAUTROU, Jean**
**21, avenue Gambetta**
**F-94100 Saint Mande (FR)**
Inventeur: **MEYER, Dominique**
**23, rue du Tage**
**F-75013 Paris (FR)**

(74) Mandataire: **Le Guen, Gérard et al**
**CABINET LAVOIX 2, place d'Estienne d'Orves**
**F-75441 Paris Cédex 09 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 263 861 B1

Courier Press, Leamington Spa, England.

**Description**

La présente invention concerne un nouveau sel du complexe gadolinium(III)—DOTA et ses applications au diagnostic comme agent de contraste en imagerie par résonance magnétique (IRM) et en radiologie paqr rayons X.

Le DOTA est l'acide 1,4,7,10 tétraazacyclododécane-N,N',N'',N'''-tétraacétique. Le complexe H[Gd-DOTA] a été mentionné par J. F. Desreux dans Inorg. Chem. 19, 1319, 1980. Par ailleurs, dans FR—A—2 539 996, on a mentionné la possibilité d'utiliser comme agents de diagnostic le sel de sodium et le sel de N-méthylglucamine de ce complexe.

On a maintenant trouvé un nouveau sel de ce complexe qui présente de façon inattendue un toxicité particulièrement faible et en particulier nettement plus faible que celle du sel de N-méthylglucamine.

La présente invention a aussi pour objet le sel de lysine du complexe gadolinium(III)—DOTA.

Ce sel peut être représenté par la formule:

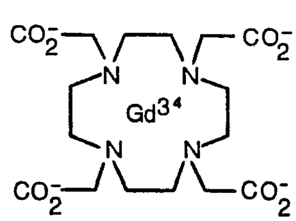

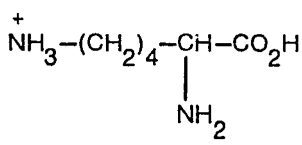

Le sel selon l'invention peut être utilisé comme agent de contraste en imagerie par résonance magnétique et en radiologue par rayons X.

La présente invention a donc également pour objet des compositions de diagnostic, administrables à l'homme, qui comprennent une quantité efficace du sel de lysine du complexe gadolinium(III)—DOTA.

Les compositions selon l'invention peuvent être administrées à l'homme par voie parentérale (notamment vasculaire ou lymphatique), sous arachnoïdienne, orale ou intra-bronchique.

Pour l'administration par voie parentérale ou orale les compositions selon l'invention peuvent être notamment des solutions du sel dans un solvant aqueux physiologiquement acceptable, ces solutions contenant notamment de $10^{-5}$mole/l à 1 mole/l du sel. La dose administrable peut être généralement de $10^{-5}$ à $10^{-3}$ mole/kg.

Pour l'administration par voie intra-bronchique les compositions peuvent être des aérosols ou des suspensions.

Les exemples suivants illustrent la préparation du sel l'invention et compositions contenant ce sel.

1. Préparation du complexe H[GdDOTA], $H_2O$

40,45 g de DOTA (0,1 mole) sont ajoutés sur une suspension composée de 18,13 g d'oxyde de gadolinium $Gd_2O_3$ (0,05 mole) dans 400 cm³ d'eau distillée.

Cette suspension est portée à 70°C sous agitation pendant 20 heures, filtrée sur filtre 0,45 µm puis évaporée à sec. La poudre résultante est reprise dans l'éther éthylique, filtrée puis séchée sous vide.

Analyse ($C_{16}H_{25}N_4O_8Gd$, $H_2O$)

| | C% | H% | N% |
|---|---|---|---|
| Calculé | 33,37 | 4,71 | 9,72 |
| Résultats | 33,31 | 4,86 | 9,82 |

2. Préparation d'une solution de sel de lysine du complexe H[GdDOTA], $H_2O$

27,93 g du complexe H[GdDOTA] sont dispersés dans une solution aqueuse de lysine (7,91 g) à 20% en poids, au moyen d'une agitation magnétique.

Le volume est ajusté à 100 cm³ par ajout d'eau distillée afin d'obtenir une solution à 0,5 mole/l.

La solution est filtrée sure filtre, 0,45 µm puis stérilisée à 120°C pendant 20 minutes.

Charactéristiques physicochimiques de la solution

*Dosage de gadolinium total* (déterminé par spectrométrie d'absorption atomique)

$$[Gd] = 0{,}501 \text{ mole/l}$$

*Dosage des traces de gadolinium non complexé* Dosage complexométrique au xylenol orange

$$[GD^{3+}] < 0{,}01\%$$

*Détermination du pH*

Avant stérilisation pH = 7,6

Après stérilisation pH = 7,7

Aucune variation après 3 mois à température ambiante.

*Densité à 20°C*

D = 1,1664

*Osmolalité obtenue par cryoscopie à 20°C*

1400 mosm/kg

On donnera ci-après des résultats mettant en évidence la toxicité particulièrement faible du sel selon l'invention.

*Toxicité aiguë par voie intraveineuse*

La toxicité aiguë a été déterminée sur des souris femelles (poids 20 à 23 g) de souche Swiss OF1 par injection intraveineuse rapide (2 ml $mn^{-1}$) selon la méthode de calcul définie par Reed et Muench dans la méthode quantale cumulative AMJ.HYG, *27*, 493, 1938. 5 animaux sont utilisés par dose avec un minimum de 3 doses par essai, ces doses suivent une progression géométrique. La toxicité ($DL_{50}$) est notée 7 jours après l'injection.

| Complexe | Sel | Concentration de la solution | Volumes injectés | Doses injectées | $DL_{50}$ mmoles/kg |
|---|---|---|---|---|---|
| $H[GdDOTA]H_2O$ | N.méthylglucamine | 0,48 mole/l | 20 à 31,6 ml, $kg^{-1}$ | 8, 12,9 mmoles $kg^{-1}$ | 10,9 |
| $H[GdDOTA]H_2O$ | Lysine | 0,501 mole/l | 31,05 à 34,15 ml, $kg^{-1}$ | 15,5 à 17,1 mmoles $kg^{-1}$ | 16,6 |
| 2H[GdDTPA] | N.méthylglucamine | 0,5 mole/l | 8,16 à 10 ml, $kg^{-1}$ | 4,8 à 5 mmoles $kg^{-1}$ | 4,45 |
| 2H[GdDTPA] | Lysine | 0,5 mole/l | 10 à 13,55 ml, $kg^{-1}$ | 5 à 6,7 mmoles $kg^{-1}$ | 5,94 |

Ces résultats mettent en évidence une amélioration beaucoup plus nette dans le cas du complexe GdDOTA quand on passe du sel de N-méthylglucamine au sel de lysine que dans le cas du complexe GdDTPA (voir EP—A—0 071 564), ce qui montre bien le caractère inattendu de la toxicité particulièrement faible du sel selon l'invention.

**Revendications**

1. Le sel de lysine du complexe de gadolinium (III) de l'acide 1,4,7,10-tétraazacyclododécane-N,N',N'',N'''-tétraacétique.

2. Composition de diagnostic administrable à l'homme, comprenant une quantité efficace du sel selon la revendication 1.

3. Composition selon la revendication 2, caractérisée en ce qu'elle est constituée par une solution du sel dans un solvant aqueux physiologiquement acceptable.

4. Composition selon la revendication 3, caractérisé en ce qu'elle contient de $10^{-5}$ mole/l à 1 mole/l du sel.

**Patentansprüche**

1. Lysin-Salz des Gadolinium-Komplex (III) der 1,4,7,10-Tetraazacyclododecan-N,N',N'',N'''-tetraessigsäure.

2. Diagnostik-Zusammensetzung zur Verabreichung an Menschen, die eine wirksame Menge des Salzes nach Anspruch 1 enthält.

3. Zusammensetzung nach Anspruch 2, dadurch gekennzeichnet, daß sie aus einer Lösung des Salzes in einem physiologisch akzeptablen wässrigen Lösungsmittel besteht.

4. Zusammensetzung nach Anspruch 3, dadurch gekennzeichnet, daß sie $10^{-5}$ mol/l bis 1 mol/1 des Salzes enthält.

**Claims**

1. The lysine salt of the gadolinium (III) complex of 1,4,7,10-tetra-azacylcododecane-N,N',N'',N'''-tetraacetic acid.

2. Diagnostic composition which can be administered to man, comprising an effective amount of the salt according to claim 1.

3. Composition according to claim 2, characterized in that it is constituted by a solution of the salt in a physiologically acceptable aqueous solvent.

4. Composition according to claim 3, characterized in that it contains from $10^{-5}$ mole/l to 1 mole/l of the salt.